# EUROPEAN PATENT APPLICATION

(11) **EP 3 269 337 A1**
(43) Date of publication of application: **17.01.2018**
(21) Application number: 16761429.6
(22) Date of filing: 12.02.2016
(51) Int. Cl.: A61F 2/966

(54) **SELF-EXPANDING STENT DELIVERY SYSTEM**

(30) Priority: 11.03.2015 JP 2015048759
(71) Applicant: Terumo Kabushiki Kaisha, Tokyo 151-0072 (JP)
(72) Inventor: OYAMA, Kenji, Fujinomiya-shi Shizuoka 418-0015 (JP); OHASHI, Kazutoshi, Fujinomiya-shi Shizuoka 418-0015 (JP)
(74) Representative: Casalonga
(86) International application number: PCT/JP2016/054155
(87) International publication number: WO 2016/143457

(57) **Abstract**

[Object]

To provide a self-expandable stent delivery system configured to be capable of moving a pull wire toward a distal side and toward a proximal side to achieve a further improvement of the operability.

[Solving Means]

An operation unit 100 includes a holding portion 200 that holds pull wires 50a, 50b so that the pull wires 50a, 50b are movable toward the distal side and toward the proximal side, and a switching portion 300 configured to be capable of switching between limitation of movement of the pull wires held by the holding portion toward the distal side and release of the limitation.

## Description

### Technical Field

The present invention relates to a self-expandable stent delivery system.

### Background Art

A stent is generally used for a treatment that expands a lesion portion, such as a stenosed site and a clogged portion, generated in body lumens such as blood vessels, bile ducts, trachea, esophagus, and urethra.

The stents include a type which is expanded by a balloon having a stent mounted thereon (balloon expandable stent) and a type which expands by itself by removing a member which restricts expansion from the outside (self-expandable stent).

The self-expandable stent expands by itself without constraint and thus does not require an expanding operation such as that required for the balloon expandable stent. The self-expandable stent having a flexibility compared to stents that do not expand by themselves may be applied to lesion portions having a meandering shape or a curved shape, and are widely used in medical sites. When indwelling the self-expandable stent as described above in a lesion portion, a self-expandable stent delivery system for delivering the self-expandable stent to the lesion portion may be used.

For example, Patent Literature 1 discloses a self-expandable stent delivery system configured to deliver a self-expandable stent in a state of being accommodated at a distal side of a catheter having an inner tube and an outer tube disposed in a periphery of the inner tube into a body lumen, move the outer tube to a proximal side by pulling a pull wire fixed to the outer tube to the proximal side by an operation on the operator's side, discharge the self-expandable stent from the catheter and expanding the self-expandable stent so that the self-expandable stent is indwelled at the lesion portion.

In the case where a configuration in which the outer tube is moved by a pushing and pulling operation of the pull wire as described above is employed, when the pull wire is pulled toward the operator's side (proximal side) by an operation unit, a pull force may act reversely to pull the pull wire toward an opposite side (distal side) to the pulling direction and thus the outer tube may inadvertently be moved. Therefore, in the self-expandable stent delivery system described above, occurrence of inadvertent movement of the outer tube is prevented by imposing a limitation on rewinding (feeding) of the pull wire toward the distal side by imposing a limitation on the movement of the pull wire toward the distal side.

### Citation List

### Patent Literature

Patent Literature 1: International Publication No.2010/093017

### Summary of Invention

### Technical Problem

However, once the pull wire is pulled, the operation of moving the pull wire toward the distal side is forcedly limited, and thus the pull wire cannot be fed toward the distal side again. For example, when indwelling the self-expandable stent in the body lumen, the pull wire may be pulled in a state in which the self-expandable stent is positioned in the vicinity of an indwelling position and then the pull wire may be moved back toward the distal side for adjusting the indwelling position (discharging position) again to achieve a state in which the self-expandable stent is accommodated in the outer tube again, and then subsequent operation may be performed continuously. However, in the case of the self-expandable stent delivery system of the related art, such an operation can hardly be achieved. For example, if the operation unit is capable of moving the pull wire reversibly toward the distal side and toward the proximal side when performing such an operation, the operability of the self-expandable stent delivery system will be further improved.

Accordingly, in order to solve the above-described problem, it is an object of the present invention to provide a self-expandable stent delivery system capable of reversibly moving a pull wire toward a distal side and toward a proximal side to achieve an improvement of the operability.

### Means for Solving the Problem

A self-expandable stent delivery system of the present invention for achieving the above-described object includes: an inner tube provided with a guide wire lumen to which a guide wire is to be inserted; a self-expandable stent disposed around a distal side of the inner tube in a state of being compressed radially inward when being inserted into a body lumen and configured to be expandable outward to restore a shape before being compressed when being indwelled in the body lumen; an outer tube configured to be capable of accommodating the self-expandable stent in an inner lumen thereof by being disposed on an outer surface side of the inner tube and discharging the self-expandable stent accommodated in the inner lumen by being moved toward the proximal side with respect to the inner tube; a pull wire configured to be capable of pulling the outer tube toward the proximal side; and an operation unit that operates advancing and retracting movement of the pull wire. The operation unit includes: a holding portion configured to hold the pull wire so that the pull wire is movable toward the distal side and toward the proximal side; and a switching portion configured to be capable of switching the pull wire held by the holding portion between limitation of movement toward the distal side and release of the limitation.

### Advantageous Effects of Invention

According to the self-expandable stent delivery system configured as described above, the pull wire may not only be moved toward the proximal side by being pulled, but also be moved toward distal side again even after having been moved once toward the proximal side. Accordingly, a further improvement of the operability is achieved.

### Brief Description of Drawings

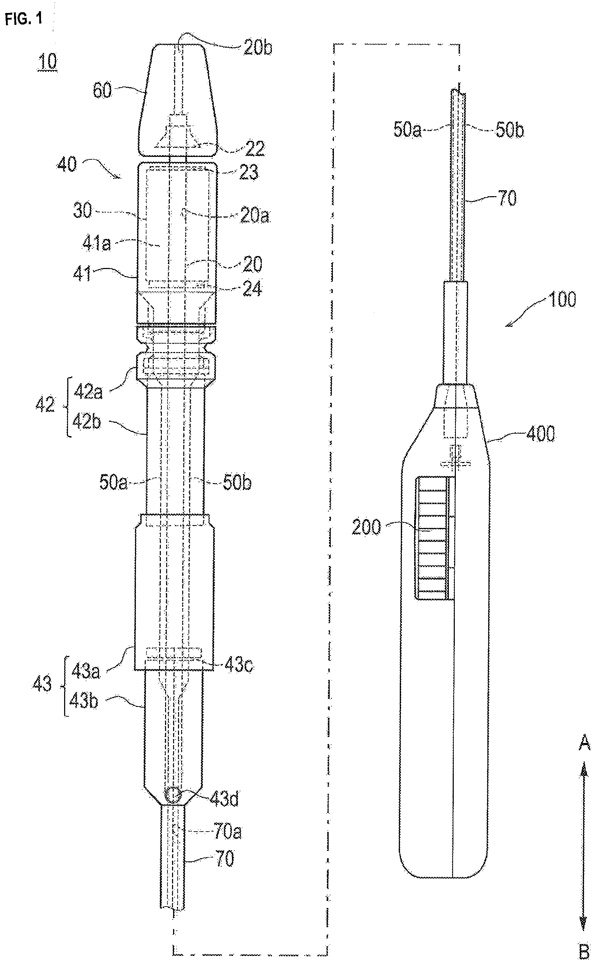

[Fig. 1] Fig. 1 is a general configuration drawing illustrating a self-expandable stent delivery system according to a first embodiment.

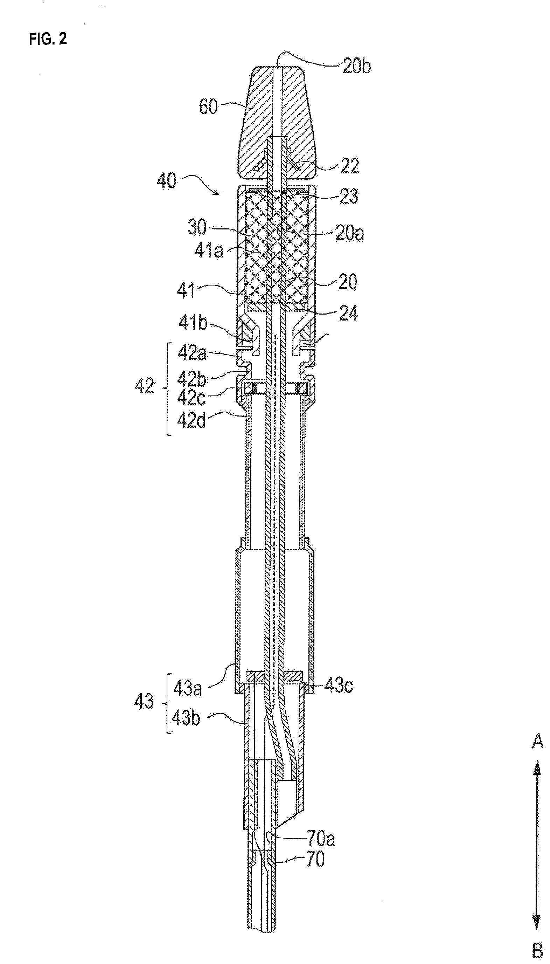

[Fig. 2] Fig. 2 is a cross-sectional view of a distal side portion of the self-expandable stent delivery system according to the first embodiment.

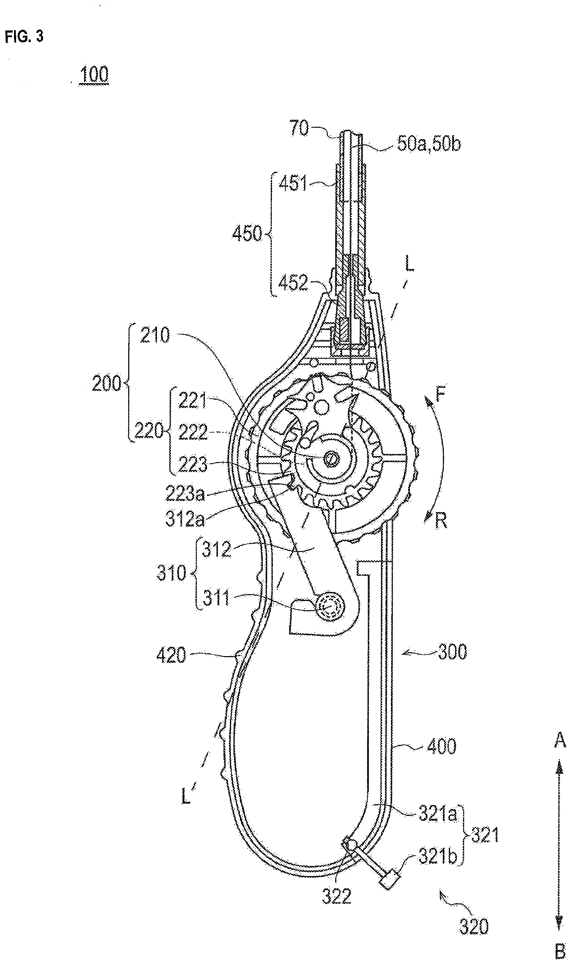

[Fig. 3] Fig. 3 is an explanatory drawing illustrating an internal structure of an operation unit of the self-expandable stent delivery system according to the first embodiment.

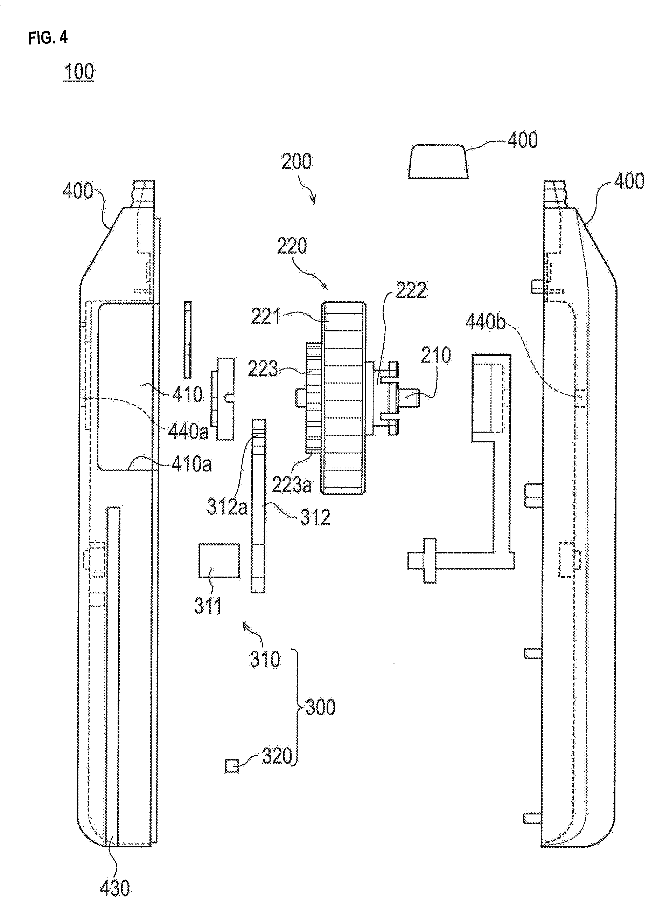

[Fig. 4] Fig. 4 is an exploded front view of the operation unit of the self-expandable stent delivery system according to the first embodiment.

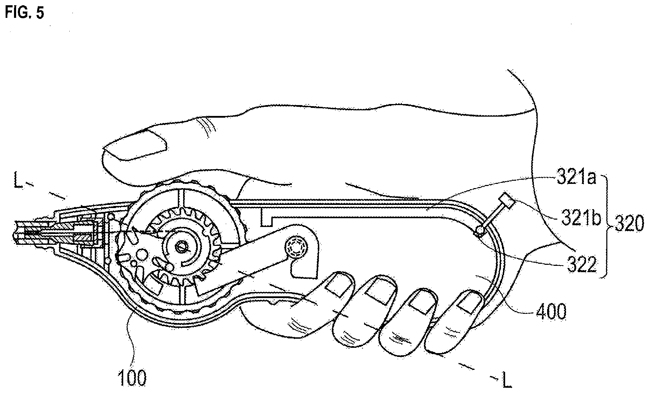

[Fig. 5] Fig. 5 is a drawing illustrating a state in which the operation unit of the self-expandable stent delivery system according to the first embodiment is gripped.

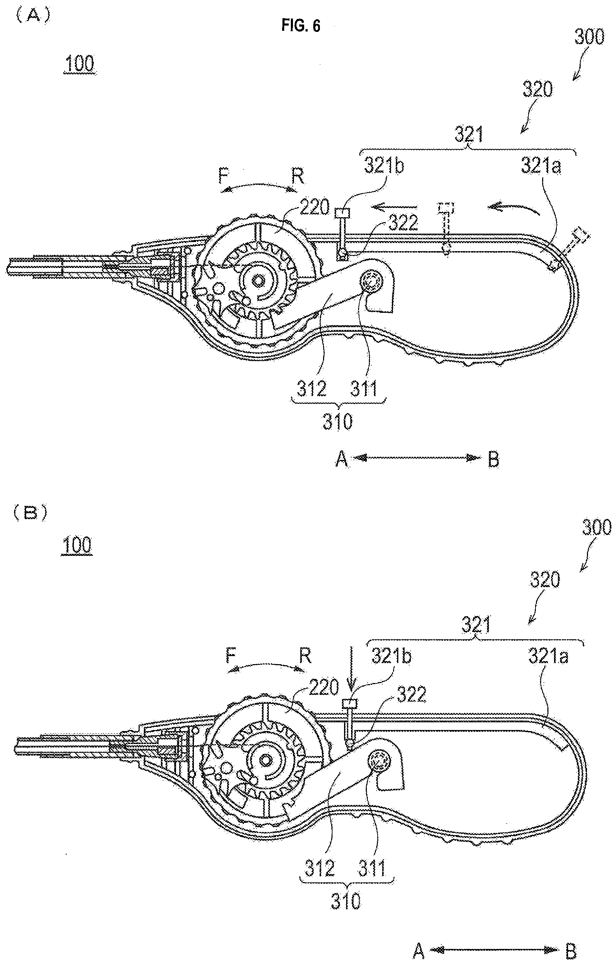

[Fig. 6] Fig. 6 illustrates explanatory drawings of the operation unit of the self-expandable stent delivery system according to the first embodiment, in which Fig. 6(A) illustrates a state in which an engagement release portion is moved to the distal side, and Fig. 6(B) illustrates a state in which an engagement release portion applies a pressing force to an engaging portion.

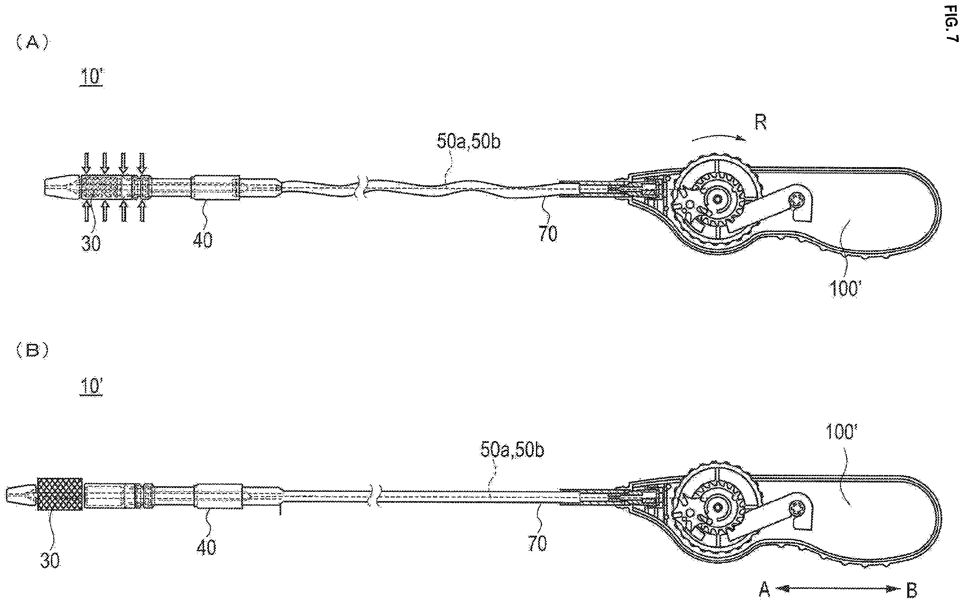

[Fig. 7] Fig. 7 illustrates drawings of a self-expandable stent delivery system according to a comparative example, in which Fig. 7(A) illustrates a state in which a pull wire insertion tube is bent when the pull wire is pulled and Fig. 7(B) illustrates a state in which the self-expandable stent is released when removing the pull wire insertion tube.

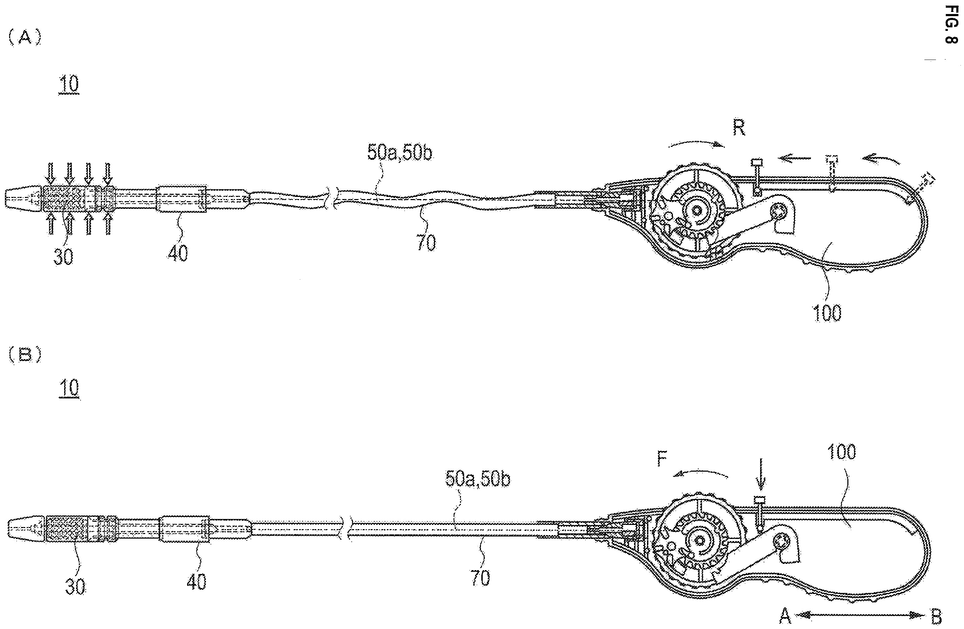

[Fig. 8] Fig. 8 illustrates drawings of the self-expandable stent delivery system according to the first embodiment, in which Fig. 8(A) is a state in which the pull wire insertion tube is bent when the pull wire is pulled, and Fig. 8(B) is a state in which the bending of the pull wire insertion tube is released by feeding the pull wire to the distal side.

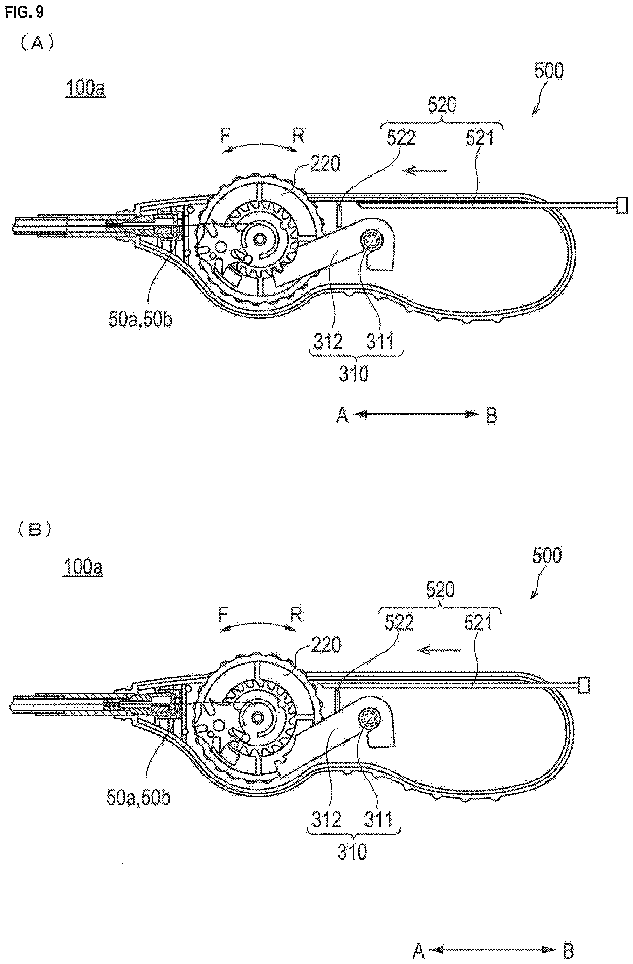

[Fig. 9] Fig. 9 illustrates explanatory drawings of an operation unit of a self-expandable stent delivery system according to a second embodiment, in which Fig. 9(A) illustrates a state in which an engagement release portion is moved to the distal side, and Fig. 9(B) illustrates a state in which the engagement release portion applies a pressing force to an engaging portion.

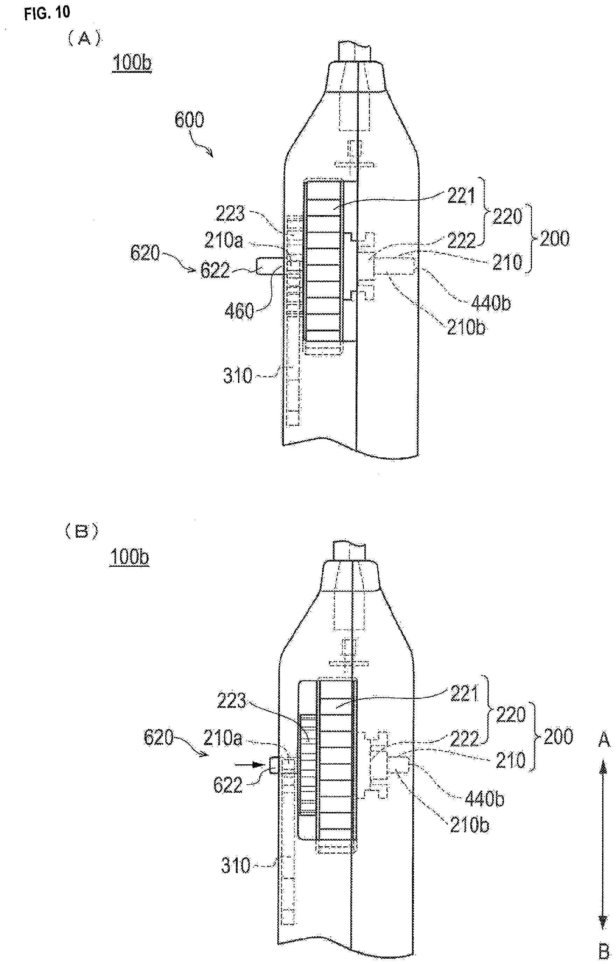

[Fig. 10] Fig. 10 illustrates explanatory drawings of an operation unit of a self-expandable stent delivery system according to a third embodiment, in which Fig. 10(A) illustrates a state in which an engagement release portion is moved to the distal side, and Fig. 10(B) illustrates a state in which the engagement release portion applies a pressing force to an engaging portion.

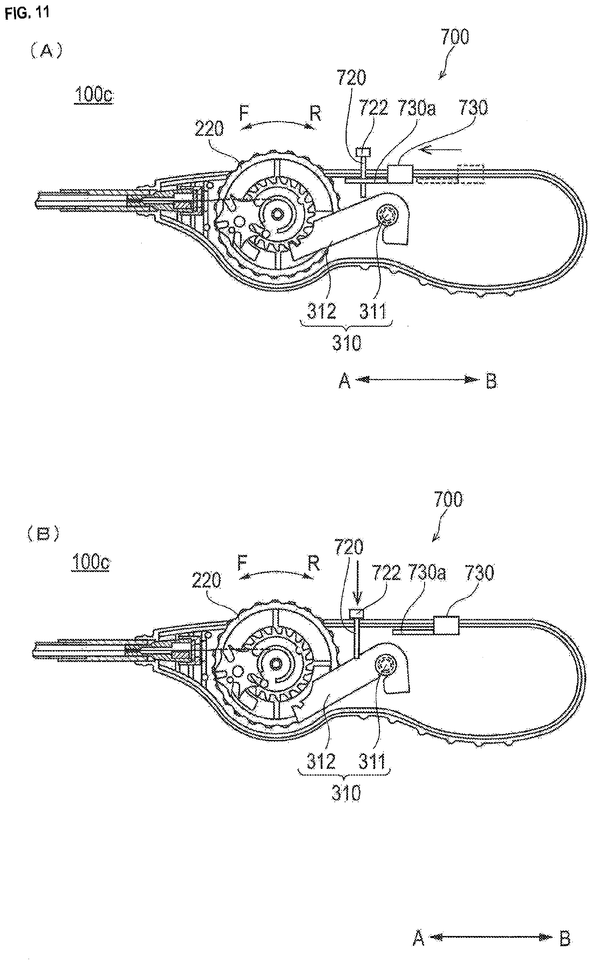

[Fig. 11] Fig. 11 illustrates explanatory drawings of an operation unit of a self-expandable stent delivery system according to a fourth embodiment, in which Fig. 11(A) illustrates a state of being restricted by a locking portion, and Fig. 11(B) illustrates a state in which restriction by the locking portion is released.

### Description of Embodiments

Referring now to the attached drawings, embodiments of the present invention will be described. It should be noted that the following description is not intended to limit the technical scope or significance of terms described in Claims. Dimensional ratios of the drawings are exaggerated for the convenience of description and may be different from actual ratios.

### First Embodiment

Fig. 1 is a general configuration drawing illustrating a self-expandable stent delivery system 10 (hereinafter referred to as a "stent delivery system 10") according to a first embodiment. Fig. 2 is a cross-sectional view of a distal side portion of the stent delivery system 10 according to the first embodiment. Fig. 3 is an explanatory drawing illustrating an internal structure of an operation unit 100 of the stent delivery system 10 according to the first embodiment. Fig. 4 is an exploded front view of the operation unit 100 of the stent delivery system 10 according to the first embodiment. Fig. 5 is a drawing illustrating a state in which the operation unit 100 according to the first embodiment is gripped. Fig. 6 illustrates explanatory drawings of the operation unit 100 according to the first embodiment, in which Fig. 6(A) illustrates a state in which an engagement release portion 320 is moved to a distal side, and Fig. 6 (B) illustrates a state in which the engagement release portion 320 applies a pressing force to an engaging portion 310. Fig. 7 illustrates drawings of a self-expandable stent delivery system 10' (hereinafter, referred to as a stent delivery system 10') according to a comparative example, in which Fig. 7(A) illustrates a state in which a pull wire insertion tube 70 is bent when the pull wire is pulled and Fig. 7(B) illustrates a state in which the self-expandable stent 30 (hereinafter, referred to as a "stent 30") is released when removing the pull wire insertion tube 70. Fig. 8 illustrates drawings of the stent delivery system 10 according to the first embodiment, in which Fig. 8(A) is a state in which the pull wire insertion tube 70 is bent when the pull wire is pulled, and Fig. 8(B) is a state in which the bending of the pull wire insertion tube 70 is released by feeding the pull wire to a distal side.

As illustrated in Fig. 1, the stent delivery system 10 according to the embodiment includes: an inner tube 20 to which a guide wire is to be inserted, the stent 30 disposed in the periphery of a distal side of the inner tube 20; an outer tube 40 disposed on an outer surface side of the inner tube 20; pull wires 50a, 50b that are capable of pulling the outer tube 40 to a proximal side; a distal end member 60 disposed on a distal-most end; the pull wire insertion tube 70 to which the pull wires 50a, 50b are inserted; and an operation unit 100 that operates advancing and retracting movement of the pull wires 50a, 50b. It should be noted that in the specification, a side which is inserted into a lumen in a living body is referred to as a distal side (a direction indicated by an arrow A in the drawings), and a side at which the operation unit 100 is provided and which comes to an operator's side is referred to as a proximal side (a direction indicated by an arrow B in the drawing).

The inner tube 20 is formed of a tube-shaped body having a guide wire lumen 20a that penetrates therethrough from a distal end to a proximal end as illustrated in Fig. 2. A guide wire (not illustrated) that guides the stent delivery system 10 to a lesion area in the body lumen is inserted to the guide wire lumen 20a.

The distal end member 60 is disposed at a distal-most end of the inner tube 20. The distal end member 60 is fixed to a distal end portion of the inner tube 20 with a stopper 22. The stopper 22 is embedded in the distal end member 60 and prevents the distal end member 60 from coming apart. The stopper 22 preferably formed of a metal (for example, stainless steel) . The distal end member 60 has a gradually tapered shape toward a distal end, and is formed to be inserted easily into the body lumen. The distal end member 60 is provided with an opening 20b at a distal end thereof. It should be noted that the distal end member 60 may be formed of a member separate from the inner tube 20, and may be integrally formed of the same member as the inner tube 20.

A proximal side of the inner tube 20 is formed to be obliquely inclined toward the proximal side and is provided so as to capable of communication with a guide wire lead-out hole 43d of the outer tube 40 described later as illustrated in Fig. 2. Accordingly, guiding of the guide wire is facilitated.

Preferably, a material that forms the inner tube 20 is a flexible material. For example, polyolefin such as polyethylene and polypropylene, polyester such as polyamide, polyamide elastomer and polyethylene terephthalate, polyester elastomer, fluorinated polymer such as ETFE, PEEK, and polyimide are preferably used. Among the resins described above, resins having especially thermoplastic property are preferably used.

Preferably, a material that forms the distal end member 60 is a flexible material. For example, synthetic resin-based elastomers such as olefin-based elastomer, polyamide elastomer, styrene-based elastomer, polyurethane, urethane-based elastomer, and fluorine resin-based elastomer, rubbers including synthetic rubbers such as urethane rubber, silicone rubber, and butadiene, and natural rubber such as latex rubber may be used.

The stent 30 is a self-expandable stent. As indicated by a dot-and-dash line in Fig. 2, when being inserted into the body lumen, the stent 30 is disposed in a stent housing 41a described later, in a state of being compressed radially inwardly about a longitudinal axis of the outer tube 40. When the outer tube 40 moves toward the proximal side, the stent 30 is exposed outward, and is discharged to a lesion area in the body lumen. Accordingly, the stent 30 is expanded radially outwardly and is restored to a shape before being compressed. The stent 30 has a meshed shape having a number of openings, and is formed into a substantially cylindrical shape. It should be noted that a material that may be used as a material of the stent 30 is preferably, for example, a superelastic alloy such as a Ni-Ti alloy.

The pull wires 50a, 50b are fixed respectively to a first outer tube 41 and a second outer tube 42 provided on the outer tube 40 described later, and pull the outer tube 40 toward the proximal side. Preferably, a material that forms the pull wires 50a, 50b is a material having a relatively high rigidity. For example, metals such as Ni-Ti, brass, stainless steel, and aluminum, or resins having a relatively high rigidity, for example, polyimide, vinyl chloride, or polycarbonate may be used.

The pull wire insertion tube 70 is formed into a tube shape having a pull wire lumen 70a that penetrates therethrough from a distal end to a proximal end as illustrated in Fig. 2. The pull wires 50a, 50b are inserted into the pull wire lumen 70a, and is guided to the operation unit 100. A distal portion of the pull wire insertion tube 70 is disposed in a lumen of the outer tube 40, and is fixed to a proximal portion of the inner tube 20. A proximal portion of the pull wire insertion tube 70 is fixed to the operation unit 100.

Preferably, a material that forms the pull wire insertion tube 70 is a flexible material. For example, polyolefin such as polyethylene and polypropylene, polyester such as polyamide and polyethylene terephthalate, fluorinated polymer such as ETFE, PEEK, and polyimide are preferably used. It should be noted that the pull wire insertion tube 70 may be coated with a resin having biocompatibility, especially antithrombotic on an outer surface thereof.
antithrombotic material that may be used includes, for example, a copolymer of poly-hydroxyethyl methacrylate, hydroxyethylmetaacrylate, and styrene.

The outer tube 40 is disposed on the distal side, and includes the first outer tube 41 that houses the stent 30, the second outer tube 42 that is disposed so as to be proximity to the proximal side of the first outer tube 41, and a third outer tube 43 disposed on the proximal side of the second outer tube 42 as illustrated in Fig. 1 and Fig. 2.

The first outer tube 41 includes a stent housing 41a that houses the stent 30 between the first outer tube 41 and the inner tube 20 in a state of being compressed radially inward. As illustrated in Fig. 2, the inner tube 20 is provided with a distal side movement limiting portion 23 that attaches a distal side of the stent 30 to impose a limitation on the movement toward the distal side and a proximal side movement limiting portion 24 that attaches a proximal side of the stent 30 to impose a limitation on the movement toward the proximal side, both fixed to the outer surface thereof. The distal side and proximal side movement limiting portions 23, 24 are formed around a longitudinal axis of the outer tube 40 into an annular shape. The stent housing 41a is formed of a portion surrounded by the proximal side movement limiting portion 24, the distal side movement limiting portion 23, and the first outer tube 41.

After the stent housing 41a is arranged on a lesion area and then the first outer tube 41 is moved to the proximal side with respect to the inner tube 20. At this time, a frictional force that makes an attempt to move toward the proximal side in association with the movement of the first outer tube 41 is applied to the stent 30. However, the stent 30 attaches the proximal side movement limiting portion 24 and thus is limited from moving toward the proximal side. Accordingly, the stent 30 can be discharged at the lesion area without being moved from the lesion area where the stent 30 is placed. The distal side movement limiting portion 23 includes at a proximal portion thereof a tapered surface that tapers towards the proximal side. Therefore, collection of the stent delivery system 10 after the discharge of the stent 30 is facilitated without being hindered by the distal side movement limiting portion 23 when discharging the stent 30.

As illustrated in Fig. 2, the first outer tube 41 is not fixed to the inner tube 20. Therefore, the first outer tube 41 is movable relatively with respect to the inner tube 20 in a direction of the longitudinal axis of the outer tube 40. The first outer tube 41 includes a fixed portion 41b to which distal ends of the pull wires 50a, 50b are fixed. The pull wires 50a, 50b are fixed to the fixed portion 41b by an adhesive agent. Epoxy resins, UV-curable resins, cyanoacrylate resins and the like may be preferably used as the adhesive agent.

In addition, the outer surface of the first outer tube 41 is preferably treated to provide lubricating property. As such a treatment, for example, a method of coating or fixing hydrophilic polymer such as polyhydroxyethyl methacrylate, polyhydroxyethyl acrylate, and polyvinyl pyrrolidone is exemplified. Alternatively, the inner surface of the first outer tube 41 may be coated or fixed with the above-described substances in order to achieve preferable sliding property of the stent 30. The first outer tube 41 may be a combination having a two-layer structure (for example, polyamide on the outer surface and PTFE on the inner surface) as described above.

Preferably, a material that forms the first outer tube 41 is a resin having a flexibility, kink resistance, elasticity and the like. For example, polyester such as polyethylene, polypropylene, polyamide, and polyethylene terephthalate, fluorinated polymer such as polyimide, PTFE, and ETFE, and thermoplastic elastomer, and the like are used. It should be noted that although the first outer tube 41, the second outer tube 42, and the third outer tube 43 are formed of the same material in the embodiment, the present invention is not limited thereto and may be formed of different materials respectively.

As illustrated in Fig. 2, the second outer tube 42 includes a distal side cylindrical portion 42a having two tube-shaped bodies having different outer diameters, a reduced diameter portion 42b, a ring-shaped member 42c for fixing pull wires 50a, 50b, and a main body portion 42d. The second outer tube 42 is movable toward the proximal side together with the first outer tube 41 by being pulled by the pull wires 50a, 50b. The second outer tube 42 is not fixed to the first outer tube 41.

The second outer tube 42 is provided in the lumen thereof with the reduced diameter portion 42b that restricts the movement of the ring-shaped member 42c toward the distal side thereof on the distal side of a portion where the ring-shaped member 42c is disposed. The reduced diameter portion 42b imposes a limitation on the movement of the ring-shaped member 42c toward the distal side by abutment of the ring-shaped member 42c therewith when the ring-shaped member 42c moves toward the distal side.

Since the ring-shaped member 42c is disposed in a loosely fitted manner with respect to the distal side cylindrical portion 42a, the ring-shaped member 42c is rotationally movable in a circumferential direction of the outer tube and is also movable in the direction of the longitudinal axis of the outer tube 40 by an amount corresponding to the gap. The pull wires 50a, 50b are fixed to the ring-shaped member 42c by an adhesive agent. Preferably, a material that forms the ring-shaped member 42c is a material having a relatively high rigidity. For example, a metal and a resin may be used and, specifically, a metal may preferably be used.

The third outer tube 43 includes a distal side tube 43a having an inner diameter larger than that of the main body portion 42d of the second outer tube 42, and a proximal side tube 43b fixed to a proximal side of the distal side tube 43a as illustrated in Fig. 2.

The distal side tube 43a is not fixed to the main body portion 42d, and is accommodated by sliding the main body portion 42d towards the proximal side. The distal side tube 43a is provided with the second outer tube movement limiting portion 43c on the proximal side thereof. The second outer tube 42 is movable toward the proximal side until attaching the second outer tube movement limiting portion 43c, and further movement toward the proximal side is limited.

In the embodiment, the distal side tube 43a of the third outer tube 43 accommodates the main body portion 42d of the second outer tube 42. However, the invention is not limited thereto, and a configuration in which the main body portion 42d may be accommodated in the distal side tube 43a by a sliding movement with a configuration in which an inner diameter of the main body portion 42d is larger than the outer diameter of the distal side tube 43a.

The proximal side tube 43b includes a guide wire lead-out hole 43d that protrudes and opens in a radial direction and obliquely outward of the third outer tube 43 as illustrated in Fig. 2. The guide wire lead-out hole 43d is provided so as to be capable of communicating with the guide wire lumen 20a of the inner tube 20, and may lead out the guide wire to the outside of the outer tube 40. The pull wire insertion tube 70 is fixed to the lumen of the proximal side tube 43b.

The operation unit 100 is fixed to the proximal end of the pull wire insertion tube 70 to which the pull wires 50a, 50b are inserted as illustrated in Fig. 1. The operation unit 100 includes a holding portion 200 that holds the pull wires 50a, 50b so that the pull wires 50a, 50b are movable toward the distal side and toward the proximal side, a switching portion 300 configured to be capable of switching between the limitation of the movement of the pull wires 50a, 50b held by the holding portion 200 toward the distal side and the release of the limitation, and an accommodation unit 400 that accommodates the holding portion 200 and the switching portion 300, as illustrated in Fig. 3.

The holding portion 200 includes a rotating shaft 210 supported at both ends thereof by the accommodation unit 400, and a rotating member 220 configured to be capable of moving rotationally about the rotating shaft 210 and to wind and feed the pull wires 50a, 50b in association with the rotary movement as illustrated in Fig. 3 and Fig. 4.

The rotating shaft 210 is provided so as to be substantially orthogonal to the direction of advancing and retracting movement of the pull wires 50a, 50b. The rotating shaft 210 may have a rolling bearing such as a ball bearing and a roller bearing and a sliding bearing containing working fluid using oil, air and the like. Metals such as carbon alloy steel and stainless steel, resin, and the like may be used to form the rotating shaft 210.

The rotating member 220 includes a disc-shaped rotating roller 221 configured to move rotationally about the rotating shaft 210 and having an irregular shape on a circumferential outer surface, a disc-shaped winding shaft portion 222 configured to rotationally move about the rotating shaft 210 in the same manner and wind and feed the pull wires 50a, 50b, and a gear portion 223 that is capable of restricting and releasing the limitation of the rotary movement of the rotating roller 221.

The rotating roller 221 includes a portion partly protruding outward of the accommodation unit 400. Hereinafter, in the operation unit 100, a side of the operation unit 100 where the rotating roller 221 protrudes is referred to as an upper side and the opposite side is referred to as a lower side. The rotating roller 221 is rotationally movable about the rotating shaft 210 by a user (operator) performing an operation for rotationally moving the protruding portion clockwise along a direction of an arrow R (a direction of winding the pull wires 50a, 50b) or counterclockwise (a direction of feeding the pull wires 50a, 50b) along a direction indicated by an arrow F.

The rotating roller 221 has an irregular shape on the circumferential outer surface thereof. The irregular shape on a surface portion with which the user (operator) may touch when operating the rotating roller 221 serves as a slip resistance, and thus an operation of rotary movement is facilitated. The irregular shape is formed, for example, by a plurality of projections disposed at substantially regular intervals along the direction of advancing and retracting movement of the pull wires 50a, 50b on a circumferential outer surface thereof. It should be noted that the slip-resistance function is not limited to the irregular shape, and surface treatments such as the circumferential outer surface of the rotating roller 221 may have a surface treatment such as knurling treatment, embossing treatment, high-resistance material coating, and the like.

The winding shaft portion 222 has a cylindrical shape, and proximal portions of the pull wires 50a, 50b are gripped by or fixed to the circumferential outer surface of the winding shaft portion 222. The winding shaft portion 222 is integrally formed with the rotating roller 221 on one lateral face of the rotating roller 221, and moves rotationally about the rotating shaft 210 along with the rotary movement of the rotating roller 221. When the winding shaft portion 222 moves rotationally clockwise along the direction of the arrow R, the proximal portions of the pull wires 50a, 50b gripped by or fixed to the circumferential outer surface are pulled, and the pull wires 50a, 50b are wound around the outer surface of the winding shaft portion 222 and are moved to the proximal side. After the pull wires 50a, 50b have been pulled by a predetermined amount, a repulsive force as counteraction of a tensile force applied by pulling is applied to an opposite side (distal side) from a pulling direction (proximal side). When the winding shaft portion 222 moves rotationally counterclockwise along a direction indicated by the arrow F, the pull wires 50a, 50b are rewound from the winding shaft portion 222 and move to the distal side.

The gear portion 223 has a disc shape, and is formed coaxially integrally with the rotating shaft 210 on a lateral face of the rotating roller 221 on the opposite side from the side where the winding shaft portion 222 is provided. The outer diameter of the gear portion 223 is smaller than the outer diameter of the rotating roller 221, and a surface of the gear portion 223 on the winding shaft portion 222 side is integrally formed with the rotating roller 221. The gear portion 223 is provided with a plurality of engaging teeth 223a on the circumferential outer surface at substantially regular intervals over the entire circumference along the direction of advancing and retracting movement of the pull wires 50a, 50b. The engaging teeth 223a have a shape inclining with respect to a radial direction in a direction indicated by the arrow F.
Accordingly, when engaging with the engaging portion 310 described later, the engagement is not released even though the gear portion 223 moves rotationally counterclockwise along the direction of the arrow F. However, the engagement is released to be rotationally movable when the gear portion 223 moves rotationally clockwise along the direction of the arrow R.

The rotating roller 221, the winding shaft portion 222, and the gear portion 223 are formed integrally. However, the invention is not limited thereto, and a configuration in which separate members follow and move rotationally along with the rotary movement of the rotating roller 221 may be employed. A method of transmitting the rotary movement of the rotating roller 221 may involve, for example, a gear system or a belt system. However, the invention is not limited thereto. Preferably, the material that is used for forming the rotating roller 221, the winding shaft portion 222, and the gear portion 223 is a material superior in abrasion resistance.

The switching portion 300, as illustrated in Fig. 3 and Fig. 4, includes an engaging portion 310 that restricts the rotary movement of the rotating member 220 by engaging with the rotating member 220 and an engagement release portion 320 that releases the limitation by the switching portion 300 by releasing the engagement between the rotating member 220 and the engaging portion 310.

The engaging portion 310 includes a fixed shaft 311 fixed to the accommodation unit 400 and a reverse rotation preventing member 312 that moves rotationally about the fixed shaft 311.

The reverse rotation preventing member 312 is provided so as to face engaging teeth 223a of the gear portion 223 and includes a depressed engaging portion 312a provided so as to be capable with engaging with one of the engaging teeth 223a by the one of the engaging teeth 223a being inserted thereto. The reverse rotation preventing member 312 is formed of a elastically deformable material, and is deformed by the engagement release portion 320 described later in a direction to move the engaging portion 312a away from the engaging teeth 223a of the gear portion 223 to disengage.
It should be noted that the reverse rotation preventing member 312 is described to be formed of an elastically deformable material, but is not limited thereto, and may be formed to be elastically deformable by having an elastic member such as a spring. For example, a configuration in which a spring is provided on the fixed shaft 311, and the reverse rotation preventing member 312 is rotationally movable in an elastic manner about the fixed shaft 311 is also applicable.

The engaging portion 312a includes a depression inclined with respect to the radial direction of the gear portion 223 in the direction indicated by the arrow F when engaging with one of the engaging teeth 223a of the gear portion 223. Therefore, as described above, the engagement is not released even though the gear portion 223 moves rotationally in the direction of the arrow F. However, the engagement is released to be rotationally movable when the gear portion 223 rotates clockwise along the direction of the arrow R. Accordingly, when the engaging portion 312a and the engaging teeth 223a engage, the rotary movement of the rotating member 220 in a feeding direction is limited, while the rotary movement of the rotating member 220 in a winding direction of the pull wires 50a, 50b is not limited and is allowed.
Movement of the pull wires 50a, 50b toward the distal side is preferably limited by the engagement between the rotating member 220 and the engaging portion 310.

The engagement release portion 320 includes a sliding member 321 provided in the accommodation unit 400 so as to be capable of advancing and retracting in a direction of the direction of advancing and retracting movement of the pull wires 50a, 50b, and a pressing member 322 provided so as to be movable toward and away from the engaging portion 310 and applying a pressing force in a direction to move the engaging portion 310 away from the rotating member 220 by moving toward the engaging portion 310 in association with the advancing and retracting movement of the sliding member 321.

The sliding member 321 includes a rail-shaped guide member 321a and a movable member 321b movable by sliding on the guide member 321a. The guide member 321a extends from a proximal portion of the accommodation unit 400 on the upper side along a shape of an upper surface of the accommodation unit 400 in the direction of advancing and retracting movement of the pull wires 50a, 50b, and extends further downward to the vicinity of a position above the reverse rotation preventing member 312. Accordingly, the movable member 321b is configured to be capable of advancing and retracting from the upper proximal portion of the accommodation unit 400 on the upper side to the vicinity of a position above the reverse rotation preventing member 312 along the direction of advancing and retracting movement of the pull wires 50a, 50b. It should be noted that the guide member 321a may be formed of a separate member from the accommodation unit 400, or may be provided integrally from the same member as the accommodation unit 400.

The movable member 321b includes a portion partly protruding outward of the accommodation unit 400. The movable member 321b is configured to be movable in the direction of advancing and retracting movement of the pull wires 50a, 50b by moving the protruding portion along the guide member 321a. The movable member 321b may be moved to the distal side along the guide member 321a, and then moved further downward to the vicinity of the position above the reverse rotation preventing member 312, and then returned back to the proximal side before the movement again.

The pressing member 322 is provided at an end portion of the movable member 321b opposite to the protruding portion and is configured to move toward and press the reverse rotation preventing member 312 in association with the advancing and retracting movement of the movable member 321b. The reverse rotation preventing member 312 may be deformed by pressing the reverse rotation preventing member 312. Accordingly, by moving the engaging teeth 223a of the rotating member 220 and the engaging portion 312a of the engaging portion 310 relatively away from each other, the engagement between the engaging teeth 223a and the engaging portion 312a may be released. Since the movable member 321b may be moved to the vicinity of the position above the reverse rotation preventing member 312 along the guide member 321a, a distance between the portion that presses the movable member 321b and the reverse rotation preventing member 312 is reduced. Therefore, a pressing force required for deforming the reverse rotation preventing member 312 may be reduced.

It should be noted that the pressing member 322 may be provided with a spring-shaped member having flexibility for alleviating an impact generating when pressing the reverse rotation preventing member 312. The reverse rotation preventing member 312 is elastically deformable, and thus engages the rotating member 220 again when the pressing force by the pressing member 322 is removed, so that the movement of the pull wires 50a, 50b toward the distal side is limited. Therefore, a stopper (not illustrated) that maintains a state in which the pressing member 322 presses the reverse rotation preventing member 312 may be provided.

The accommodation unit 400 is bent at a proximal side and a center portion and has a rounded shape to accommodate the holding portion 200 and the switching portion 300 as illustrated in Fig. 3 and Fig. 4. The accommodation unit 400 includes a first opening portion 410 opening at an upper surface, a finger hooking portion 420 on which a finger can be hooked when operating the pull wires 50a, 50b via the holding portion 200, a second opening portion 430 that exposes part of the engagement release portion 320, bearing portions 440a, 440b, and a coupling member 450.

The first opening portion 410 is an opening provided on the upper surface of the accommodation unit 400, and exposes part of the rotating roller 221 of the holding portion 200 to outside of the accommodation unit 400. Since the rotating roller 221 may be operated from the outside of the accommodation unit 400, the operation is facilitated. An opening edge portion 410a and the rotating roller 221 are configured not be away from each other to avoid interference, and are configured so that the accommodation unit 400 does not hinder the operation of rotary movement even when the rotating roller 221 is rotated.

The finger hooking portion 420 is formed on a lower surface of the accommodation unit 400, and has a plurality of curved portions. The size of the curved portions is formed to follow a curved surfaces of human fingers on a palm side. The finger hooking portion 420 allows the user (operator) to hook his or her fingers when gripping the accommodation unit 400 as illustrated in Fig. 5, so that stable gripping of the accommodation unit 400 is achieved. It should be noted that the number of the curved portions is preferably at least four so as to allow all fingers other than the thumb to be hooked thereon, but is not limited thereto.

The second opening portion 430 is formed at a position away from an extension line L (broken line in Fig. 3) that connects the first opening portion 410 and the finger hooking portion 420 to a proximal side, and exposes part of the movable member 321b of the engagement release portion 320 to the outside of the accommodation unit 400. Since the movable member 321b may be operated from the outside of the accommodation unit 400, the operation is facilitated. Specifically, the second opening portion 430 is opened from the proximal portion of the accommodation unit 400 on the upper side to a position above the reverse rotation preventing member 312 on the proximal side with respect to the first opening portion 410. Accordingly, as illustrated in Fig. 5, a protruding portion of the movable member 321b of the engagement release portion 320 may be disposed on the proximal portion of the accommodation unit 400 on the upper side, where the hand does not touch during the operation, so that inadvertent release of the limitation of rotary movement of the rotating member 220 by the switching portion 300 during the operation is avoided. When the release of the limitation is desired, the movable member 321b of the engagement release portion 320 is moved to the vicinity of the position above the reverse rotation preventing member 312, and is pressed against the reverse rotation preventing member 312 to release the limitation.

The two bearing portions 440a, 440b accommodate one end of the rotating shaft 210 of the rotating member 220 on the gear portion 223 side and the other end of the rotating shaft 210 on the winding shaft portion 222 side, respectively, as illustrated in Fig. 4. Accordingly, the rotating member 220 is accommodated in the accommodation unit 400 by being supported at both ends thereof.

The coupling member 450 includes a connector 451 that is interlocked with the pull wire insertion tube 70 and a seal member 452. A distal portion of the connector 451 is fixed to the proximal portion of the pull wire insertion tube 70. The seal member 452 is connected to a proximal portion of the connector 451.

Hereinafter, a method of releasing the limitation of movement of the pull wires 50a, 50b held by the holding portion 200 toward the distal side by the switching portion 300 in the operation unit 100 of the first embodiment will be described.

First of all, as illustrated in Fig. 3, before releasing the limitation of movement of the pull wires 50a, 50b toward the distal side, the engagement release portion 320 is disposed on the proximal portion of the accommodation unit 400 on the upper side. At this time, the reverse rotation preventing member 312 of the engaging portion 310 engages the engaging portion 310 of the rotating member 220, the rotary movement of the rotating member 220 to the feeding direction of the rotating member 220 (the direction indicated by the arrow F) is limited, and the movement of the pull wires 50a, 50b held by the holding portion 200 toward the distal side is limited.

When release of the limitation is desired, first of all, the movable member 321b of the engagement release portion 320 is moved along the direction of advancing and retracting movement of the pull wires 50a, 50b from the proximal portion of the accommodation unit 400 on the upper side to the position above the reverse rotation preventing member 312 on the distal side as illustrated in Fig. 6(A). Next, as illustrated in Fig. 6(B), the guide member 321a is pushed downward and then the reverse rotation preventing member 312 is pressed downward. With the pressing force, the reverse rotation preventing member 312 is deformed downward, and engagement is released by moving the engaging portion 310 relatively away from each other with respect to the gear portion 223 of the rotating member 220. In this state, as the rotating member 220 is capable of moving rotationally in the feeding direction (the direction indicated by the arrow F), the movement of the pull wires 50a, 50b toward the distal side is released from the limitation and thus is allowed. Accordingly, the limitation of movement of the pull wires 50a, 50b toward the distal side may be released by an action of moving the members away from each other, and thus simple operation is achieved. In addition, with the operation as easy as pressing the reverse rotation preventing member 312, the release of the limitation of the movement of the pull wires 50a, 50b toward the distal side may be achieved further quickly.

Figs. 7(A) and (B) illustrate a stent delivery system 10' according to a comparative example of the stent delivery system 10 according to the embodiment. The stent delivery system 10' of the comparative example is provided with the engaging portion 310 that restricts the movement of the pull wires 50a, 50b toward the distal side, but is not provided with the engagement release portion 320 that releases the limitation.

As illustrated in Fig. 7(A), when a distal side portion of the stent delivery system 10' is inserted into a stenosed site (lesion area), a load (constraint force) may be applied to the distal side portion by the stenosed site radially inward (the direction indicated by arrows in Fig. 7(A)) that presses the outer tube 40. When the inner diameter of the stenosed site is extremely narrow, the radially inward load is excessively high, and thus the movement of the outer tube 40 toward the proximal side following the pulling operation of the pull wires 50a, 50b may be hindered. At this time, if an attempt is made to forcedly pull the pull wires 50a, 50b, the distance between the outer tube 40 and an operation unit 100' is decreased, but the position of the outer tube 40 does not change. Accordingly, the pull wire insertion tube 70, to which the pull wires 50a, 50b are inserted, on the proximal side of the outer tube 40 may be distorted by an amount corresponding to the reduction of the distance between the outer tube 40 and the operation unit 100'. In such a case, when the outer tube is moved to the proximal side by making an attempt to remove the distal side portion of the stent delivery system 10', the outer tube 40 is suddenly opened from the constraint force applied thereto by the stenosed site. Accordingly, by a restoration force that tries to restore the distortion of the pull wire insertion tube 70, a force in the direction of releasing the stent 30 to the distal side of the outer tube 40 is applied to the stent 30 so that the stent 30 may be discharged at an unintended timing as illustrated in Fig. 7 (B) . The user is obliged to perform an operation for removing the distal side portion of the stent delivery system 10' while paying attention not to cause such discharge of the stent 30, and thus an increased procedure time may result.

In the stent delivery system 10 illustrated in Fig. 8, when the distal side portion is inserted into the stenosed site (lesion area) as illustrated in Fig. 8(A), a constraint force that presses the outer tube 40 radially inward at the stenosed site is applied in the same manner as the comparison example, so that the pull wire insertion tube 70 on the proximal side with respect to the outer tube 40 may be distorted. At this time, movement of the pull wires 50a, 50b to the distal side is enabled by the engagement release portion 320 that releases the limitation of the movement of the pull wires 50a, 50b toward the distal side by the engaging portion 310. As illustrated in Fig. 8(B), the state before being pulled is restored by moving the pull wires 50a, 50b toward the distal side, so that the distortion of the pull wire insertion tube 70 may be solved. Since the distal side portion may be removed from this state, removal is facilitated, and the operability is improved.

As described above, the operation unit 100 of the stent delivery system 10 according to the first embodiment includes the holding portion 200 that holds the pull wires 50a, 50b in a state of being movable toward the distal side and toward the proximal side, the switching portion 300 configured to be capable of switching between the limitation of the movement of the pull wires 50a, 50b held by the holding portion 200 toward the distal side and the release of the limitation.

According to the stent delivery system 10 configured in this manner, the pull wires 50a, 50b may not only be moved toward the proximal side by being pulled, but also be moved toward distal side again even after having been moved once toward the proximal side. Accordingly, a further improvement of the operability is achieved. In addition, discharge of the stent 30 at an unintended timing may preferably be prevented even in the case where the constraint force that presses the outer tube 40 radially inward is applied in a stenosed site or the like that is present in the body lumen, convenience at the time of use is improved.

In addition, the holding portion 200 is configured to be capable of moving rotationally and includes the rotating member 220 that winds and feeds the pull wires 50a, 50b along with the rotary movement, and the switching portion 300 includes the engaging portion 310 that restricts the rotary movement of the rotating member 220 by engaging with the rotating member 220 and the engagement release portion 320 that releases the limitation by the switching portion 300 by releasing the engagement between the rotating member 220 and the engaging portion 310.

According to the stent delivery system 10 configured as described above, the movement of the pull wires 50a, 50b toward the distal side is preferably limited by the engagement between the rotating member 220 and the engaging portion 310. The release of the limitation may easily be achieved by releasing the engagement.

The engagement release portion 320 is configured in such a manner that engagement may be released in association with the relative movement of the rotating member 220 and the engaging portion 310 away from each other.

According to the stent delivery system 10 configured as described above, the engagement release portion 320 may release the limitation of movement of the pull wires 50a, 50b toward the distal side by an action of moving the members away from each other, and thus simple operation is achieved.

In addition, the accommodation unit 400 that accommodates the holding portion 200 and the switching portion 300 is further provided. The engagement release portion 320 includes a sliding member 321 provided in the accommodation unit 400 so as to be capable of advancing and retracting in the direction of advancing and retracting movement of the pull wires 50a, 50b, and a pressing member 322 provided so as to be movable toward and away from the engaging portion 310 and applying a pressing force in the direction to move the reverse rotation preventing member 312 (the engaging portion 310) away from the rotating member 220 by moving toward the engaging portion 310 in association with the advancing and retracting movement of the sliding member 321.

According to the stent delivery system 10 configured as described above, with the operation as easy as pressing the reverse rotation preventing member 312, the release of the limitation of the movement of the pull wires 50a, 50b toward the distal side may be achieved further quickly.

In addition, the sliding member 321 includes a rail-shaped guide member 321a and a movable member 321b movable by sliding on the guide member 321a, and the pressing member 322 is provided at an end portion of the movable member 321b.

According to the stent delivery system 10 configured as described above, since the movable member 321b may be moved to the vicinity of the position above the reverse rotation preventing member 312 along the guide member 321a, a distance between the portion that presses the movable member 321b and the reverse rotation preventing member 312 is reduced. Therefore, a pressing force required for deforming the reverse rotation preventing member 312 may be reduced.

In addition, the accommodation unit 400 includes the first opening portion 410 that exposes part of the holding portion 200 to the outside, the finger hooking portion 420 on which a finger can be hooked when operating the pull wires 50a, 50b via the holding portion 200, the second opening portion 430 that exposes part of the engagement release portion 320 at a position away from the extension line L that connects the first opening portion 410 and the finger hooking portion 420 toward the proximal side.

According to the stent delivery system 10 configured as described above, the engagement release portion 320 may be disposed at a position where the hand does not touch during the operation, so that inadvertent release of the limitation of rotary movement of the rotating member 220 by the switching portion 300 during the operation is avoided.

### [Second Embodiment]

Fig. 9 illustrates explanatory drawings of an operation unit 100a of a self-expandable stent delivery system 10a (hereinafter, referred to as a "stent delivery system 10a) according to a second embodiment, in which Fig. 9(A) illustrates a state in which an engagement release portion 520 is moved to the distal side, and Fig. 9(B) illustrates a state in which the engagement release portion 520 applies a pressing force to an engaging portion 310. Referring to Fig. 9, the stent delivery system 10a according to the second embodiment will be described below.

The stent delivery system 10a of the second embodiment is different from that of the first embodiment only in the configuration of the engagement release portion 520 of the operation unit 100a, and other configurations are the same as those of the first embodiment. Description of the same configurations as the first embodiment will be omitted below. It should be noted that members having the same configuration as the first embodiment will be described with the same reference numerals.

An engagement release portion 520 according to the second embodiment includes a sliding member 521 provided in an accommodation unit 400 so as to be capable of advancing and retracting in a direction of advancing and retracting movement of pull wires 50a, 50b, and a pressing member 522 provided so as to be movable toward and away from the engaging portion 310 and applying a pressing force in a direction to move the engaging portion 310 away from a rotating member 220 by moving toward the engaging portion 310 in association with the advancing and retracting movement of the sliding member 521 as illustrated in Fig. 9.

The sliding member 521 has a rod-shaped portion extending from the proximal portion of the accommodation unit 400 on the upper side to the position above the reverse rotation preventing member 312 along the direction of advancing and retracting movement of the pull wires 50a, 50b, and the proximal portion is configured to attach an upper end portion of the pressing member 522. In the same manner as the first embodiment, the sliding member 521 includes a portion partly protruding outward from a second opening of the accommodation unit 400. The sliding member 521 is configured to be movable in the direction of advancing and retracting movement of the pull wires 50a, 50b by moving the protruding portion inward and outward.

The pressing member 522 is provided at the vicinity of a position above the reverse rotation preventing member 312. By the movement of the sliding member 521 toward the distal side, the upper side of the pressing member 522 attaches the distal side of the sliding member 521. The distal side of the sliding member 521 and the upper side of the pressing member 522 are formed so as to be slidably movable with each other, and the pressing member 522 is configured to be movable downward by the sliding movement. By the operation to push the sliding member 521, the pressing member 522 approaches the reverse rotation preventing member 312 and presses the same.

Hereinafter, a method of releasing the limitation of movement of the pull wires 50a, 50b held by the holding portion 200 toward the distal side by a switching portion 500 in the operation unit 100a of the second embodiment will be described.

As illustrated in Fig. 9(A), before releasing the limitation of the movement of the pull wires 50a, 50b toward the distal side, the sliding member 521 is disposed at a position on the proximal side that does not cause contact with the pressing member 522. At this time, the reverse rotation preventing member 312 of the engaging portion 310 engages the engaging portion 310 of the rotating member 220, the rotary movement of the rotating member 220 to the feeding direction of the rotating member 220 (the direction indicated by the arrow F) is limited, and the movement of the pull wires 50a, 50b held by the holding portion 200 toward the distal side is limited.

When the release of the limitation is desired, as illustrated in Fig. 9(B), the portion of the sliding member 521 protruding from the accommodation unit 400 is pushed to the distal side.
The distal side of the sliding member 521 and the upper side of the pressing member 522 attach with each other, then the pressing member 522 is moved downward, so that the pressing member 522 presses the reverse rotation preventing member 312. With the pressing force, the reverse rotation preventing member 312 is deformed downward, and engagement is released by moving the engaging portion 310 relatively away from each other with respect to the gear portion 223 of the rotating member 220. In this state, as the rotating member 220 is capable of moving rotationally in the feeding direction (the direction indicated by the arrow F), the movement of the pull wires 50a, 50b toward the distal side is released from the limitation and thus is allowed. Accordingly, with the operation as easy as pressing the reverse rotation preventing member 312, the release of the limitation of the movement of the pull wires 50a, 50b toward the distal side may be achieved further quickly. In addition, since the limitation of the movement of the pull wires 50a, 50b toward the distal side may be released by a single action of pushing the sliding member 521 inward, the releasing operation may be performed in a short time.

As described above, the operation unit 100a of the stent delivery system 10a according to the second embodiment has the accommodation unit 400 that accommodates the holding portion 200 and the switching portion 500 in the same manner as the first embodiment. The engagement release portion 520 includes a sliding member 521 provided in the accommodation unit 400 so as to be capable of advancing and retracting in the direction of advancing and retracting movement of the pull wires 50a, 50b, and a pressing member 522 provided so as to be movable toward and away from the engaging portion 310 and applying a pressing force in the direction to move the reverse rotation preventing member 312 (the engaging portion 310) away from the rotating member 220 by moving toward the engaging portion 310 in association with the advancing and retracting movement of the sliding member 521.

According to the stent delivery system 10a configured as described above, the release of the limitation of the movement of the pull wires 50a, 50b toward the distal side is easily achieved with a simple operation of pressing the reverse rotation preventing member 312 in the same manner as in the first embodiment, further improvement of the operability is achieved.

In addition, the distal side of the sliding member 521 and the upper side of the pressing member 522 are formed so as to be in slidable attachment with each other, and the pressing member 522 is configured to be movable downward by the sliding movement. According to the stent delivery system 10a configured as described above, since the limitation of the movement of the pull wires 50a, 50b toward the distal side may be released by a single action of pushing the sliding member 521 inward, the releasing operation may be performed in a short time.

### Third Embodiment

Fig. 10 illustrates explanatory drawings of an operation unit 100b of a self-expandable stent delivery system 10b (hereinafter, referred to as a "stent delivery system 10b") according to a third embodiment, in which Fig. 10(A) illustrates a state in which an engagement release portion 620 is moved to the distal side, and Fig. 10 (B) illustrates a state in which the engagement release portion 620 applies a pressing force to an engaging portion 310. Referring to Fig. 10, the stent delivery system 10b according to the third embodiment will be described below.

The stent delivery system 10b of the third embodiment is different from that of the first embodiment only in the configuration of a holding portion 200 of an operation unit 100b, the engagement release portion 620, and the bearing portion, and other configurations are the same as those of the first embodiment. Description of the same configurations as the first embodiment will be omitted below. It should be noted that members having the same configuration as the first embodiment will be described with the same reference numerals.

The holding portion 200 according to the third embodiment includes a rotating shaft 210 supported at both ends thereof by an accommodation unit 400, and a rotating member 220 configured to be capable of moving rotationally about the rotating shaft 210 and moving in the direction of the rotating shaft 210, and to wind and feed pull wires 50a, 50b in association with the rotary movement. It should be noted that the configurations of the components of the rotating member 220 of the stent delivery system 10b according to the third embodiment are the same as those of the first embodiment.

The rotating member 220 of the third embodiment is different from that of the first embodiment in being configured to be movable in the direction of the rotating shaft 210. The direction of movement in the direction of the rotating shaft 210 is not specifically limited. However, in this embodiment, the respective components move integrally toward a winding shaft portion 222 with respect to a rotating roller 221.

The structure of the rotating shaft 210 is not specifically limited. However, a configuration including a first rotating shaft 210a disposed on a gear portion 223 side and a second rotating shaft 210b disposed on the winding shaft portion 222 side is applicable. The first rotating shaft 210a and the second rotating shaft 210b have a slidable telescopic structure with respect to each other. The rotating member 220 is integrally assembled to an outer periphery of the first rotating shaft 210a, and is configured to move to retract in the axial direction together with the axial movement of the first rotating shaft 210a. The second rotating shaft 210b slides along with the movement of the first rotating shaft 210a in the axial direction and is housed in the interior of the first rotating shaft 210a. With the configuration described above, the rotating shaft 210 is capable of expanding in the axial direction.

The bearing portion that supports the rotating shaft 210 includes only a bearing portion 440b on the winding shaft portion 222 side for the rotating roller 221, and on the other side, an insertion hole 460 to which the rotating shaft 210 is inserted is formed.

The engagement release portion 620 according to the third embodiment includes a pressing member 622 capable of releasing the engagement along with a relative movement of the rotating member 220 and the engaging portion 310 away from each other in the direction of the rotating shaft 210 as illustrated in Fig. 10(A).

The pressing member 622 is provided coaxially with the rotating shaft 210 of the rotating member 220. The pressing member 622 is fixed to one end portion of the rotating shaft 210 inserted into the insertion hole 460. By pressing the pressing member 622 inward into the accommodation unit 400, the rotating shaft 210 is pushed inward of the accommodation unit 400 from a lateral face side where the pressing member 622 is provided. Accordingly, the rotating shaft 210 is contracted. At this time, the other end portion of the rotating shaft 210 does not move in the axial direction by the bearing portion of the accommodation unit 400. Accordingly, the rotating member 220 is configured to be movable in the direction of the rotating shaft 210.

Hereinafter, a method of releasing the limitation of movement of the pull wires 50a, 50b held by the holding portion 200 toward the distal side by the switching portion 600 in the operation unit 100b of the third embodiment will be described.

First of all, as illustrated in Fig. 10(A), before releasing the limitation of the movement of the pull wires 50a, 50b toward the distal side, the engaging portion 310 is disposed on a plane of rotation of the gear portion 223. At this time, the reverse rotation preventing member 312 of the engaging portion 310 engages the engaging portion 310 of the rotating member 220, the rotary movement of the rotating member 220 to the feeding direction of the rotating member 220 (the direction indicated by the arrow F) is limited, and the movement of the pull wires 50a, 50b held by the holding portion 200 toward the distal side is limited.

When the release of the limitation is desired, as illustrated in Fig. 10(B), the pressing member 622 is pressed from the lateral face side where the pressing member 622 is provided to the other lateral face side. The pressing force applied by the pressing member 622 causes the rotating shaft 210 to contract. Along with the contraction, the rotating member 220 moves to the other lateral face side. The engagement is released by relatively moving the gear portion 223 away from the engaging portion 310 of the rotating member 220 in the direction of the rotating shaft 210. In this state, as the rotating member 220 is capable of moving rotationally in the feeding direction (the direction indicated by the arrow F), the movement of the pull wires 50a, 50b toward the distal side is released from the limitation and thus is allowed. Accordingly, the plane of rotation of the gear portion 223 and the motion area of the engaging portion 310 do not overlap with each other, inadvertent release due to an elastic deformation of the reverse rotation preventing member 312 may be avoided. Therefore, a further improvement of the operability is achieved. In addition, by pulling back the pressing member 622, the limitation of the movement of the pull wires 50a, 50b toward the distal side may be restored.

As described above, the engagement release portion 620 of the stent delivery system 10b according to the third embodiment is configured to be capable of releasing the engagement in association with the relative movement between the gear portion 223 of the rotating member 220 and the engaging portion 310 away from each other in the direction of the rotating shaft 210.

According to the stent delivery system 10b configured as described above, the plane of rotation of the gear portion 223 and the motion area of the engaging portion 310 do not overlap with each other, inadvertent release due to the elastic deformation of the reverse rotation preventing member 312 may be avoided. Therefore, a further improvement of the operability is achieved.

### Fourth Embodiment

Fig. 11 illustrates explanatory drawings of an operation unit 100c of a self-expandable stent delivery system 10c (hereinafter, referred to as a "stent delivery system 10c") according to a fourth embodiment, in which Fig. 11(A) illustrates a state in which the restriction by a locking portion 730 is effected, and Fig. 11(B) illustrates a state in which the restriction by the locking portion 730 is released Referring to Fig. 11, a stent delivery system 10c according to the fourth embodiment will be described below.

The stent delivery system 10c of the fourth embodiment is different from that of the first embodiment only in the configurations of an engagement release portion 720 and the locking portion 730, and other configurations are the same as those of the first embodiment. Description of the same configurations as the first embodiment will be omitted below. It should be noted that members having the same configuration as the first embodiment will be described with the same reference numerals.

The stent delivery system 10c according to the fourth embodiment further includes the locking portion 730 that is switchable between a restriction of the releasing operation of a switching portion 700 that releases the limitation and release of the limitation. The locking portion 730 is provided on an upper side of an accommodation unit 400 so as to be slidably movable. The locking portion 730 further includes a locking member 730a that locks a pressing member 722.

The engagement release portion 720 includes a pressing member 722 capable of releasing the engagement along with a relative movement of the rotating member 220 and the engaging portion 310 away from each other in the direction of a rotating shaft 210.

Hereinafter, a method of releasing the limitation of movement of the pull wires 50a, 50b held by the holding portion 200 toward the distal side by the switching portion 700 in an operation unit 100c of the fourth embodiment will be described.

As illustrated in Fig. 11(A), before releasing the limitation of the movement of the pull wires 50a, 50b toward the distal side, the pressing member 722 is disposed at a position above the reverse rotation preventing member 312. In this state, the locking portion 730 locks the pressing member 722 by the locking member 730a, the downward movement of the pressing member 722 that presses the reverse rotation preventing member 312 is restricted. At this time, the reverse rotation preventing member 312 of the engaging portion 310 engages the engaging portion 310 of the rotating member 220, the rotary movement of the rotating member 220 to the feeding direction of the rotating member 220 (the direction indicated by the arrow F) is limited, and the movement of the pull wires 50a, 50b held by the holding portion 200 toward the distal side is limited.

When release of the limitation is desired, as illustrated in Fig. 11(B), the locking portion 730 is moved the locking member 730a away from the pressing member 722 to unlock by moving the locking portion 730 toward the proximal side so that the restriction by the locking portion 730 is released. Subsequently, the pressing member 722 is pushed downward. The pressing member 722 moves downward and the pressing member 722 presses the reverse rotation preventing member 312. With the pressing force, the reverse rotation preventing member 312 is deformed downward, and engagement is released by moving the engaging portion 310 relatively away from each other with respect to the gear portion 223 of the rotating member 220. In this state, as the rotating member 220 is capable of moving rotationally in the feeding direction (the direction indicated by the arrow F), the movement of the pull wires 50a, 50b toward the distal side is released from the limitation and thus is allowed. Accordingly, if the releasing operation is not performed on the locking portion 730, a releasing operation that releases the limitation applied by the switching portion 700 may not be performed. Therefore, irrespective of the disposition of the engagement release portion 720 of the switching portion 700 during the operation, the limitation on the rotary movement of the rotating member 220 may not be released inadvertently by the switching portion 700 during the operation, so that a further improvement of the operability is achieved.

As described above, the stent delivery system 10c according to the fourth embodiment further includes the locking portion 730 that is capable of switching between the restriction of the releasing operation of the switching portion 700 for releasing the limitation and the release of the restriction.

According to the stent delivery system 10c configured as described above, irrespective of the disposition of the engagement release portion 720 of the switching portion 700, the limitation on the rotary movement of the rotating member 220 may not be released inadvertently by the switching portion 700 during the operation, so that a further improvement of the operability is achieved.

Although the stent delivery systems 10, 10a, 10b, 10c have been described through the embodiments thus far, the present invention is not limited to the configuration described in the embodiments, and may be modified as needed based on the description of the Claims.

For example, in all of the embodiments, the pull wires 50a, 50b are wound when the rotating member 220 moves rotationally clockwise along the direction indicated by an arrow R, and the pull wires 50a, 50b are fed when the pull wires 50a, 50b is wound and move rotationally counterclockwise along the direction indicated by the arrow F. However, the invention is not limited thereto, and a configuration in which the pull wires 50a, 50b are wound when moving rotationally in the direction indicated by an arrow F and are fed when moving rotationally clockwise along the direction indicated by the arrow R is also applicable.

The holding portion 200 includes the gear portion 223, and the movement of the pull wires 50a, 50b toward the distal side is limited by the engagement between the gear portion 223 and the engaging portion 310. However, the present invention is not limited thereto. For example, a configuration provided with the winding shaft portion 222 and the engaging teeth 223a and engages the engaging portion 310 is also applicable.

As the first embodiment, the second embodiment, and the third embodiment have a configuration in which the pressing member 322 may be pressed against the engaging portion 310 once, and then restoration to the state before being pressed may be performed. However, the invention is not limited thereto, and may have a structure in which once pushed inward, the state in which the engaging portion 310 is pressed may not be released.

In addition, the configuration in which the locking portion 730 described as the fourth embodiment is applied is not limited to the corresponding embodiment, and may be applied, for example, to the stent delivery systems 10, 10a ,10b in the first embodiment, the second embodiment, and the third embodiment.

In the first embodiment, the second embodiment, and the fourth embodiment, the pressing member 322 is described to release the engagement by pressing the reverse rotation preventing member 312 downward. However, the pressing direction is not limited thereto, and the reverse rotation preventing member 312 may be pressed from any direction.

Although the outer tube 40 includes the first outer tube 41, the second outer tube 42, and the third outer tube 43, the invention is not limited thereto, and one or two outer tubes, or four or more outer tubes may be provided.

This application is based on Japanese Patent Application No. 2015-048759 filed on March 11, 2015, and the entire part of the disclosure is incorporated herein by reference.

### Reference Signs List

10, 10', 10a, 10b, 10c stent delivery system (self-expandable stent delivery system)
20 inner tube
30 stent (self-expandable stent)
40 outer tube
50a, 50b, pull wire
60 distal end member
70 pull wire insertion tube
100 ,100', 100a, 100b, 100c operation unit
200 holding portion
210 rotating shaft
220 rotating member
221 rotating roller
222 winding shaft portion
223 gear portion
223a engaging tooth
300. 500, 600 ,700 switching portion
310 engaging portion
311 fixed shaft
312 reverse rotation preventing member
312a engaging portion
320, 520, 620, 720 engagement release portion
321, 521, sliding member
321a guide member
321b movable member
322, 522, 622, 722 pressing member
730 locking portion
400 accommodation unit
410 first opening portion
420 finger hooking portion
430 second opening portion
L extension line

## Claims

1. A self-expandable stent delivery system comprising: an inner tube provided with a guide wire lumen to which a guide wire is to be inserted; a self-expandable stent disposed around a distal side of the inner tube in a state of being compressed radially inward when being inserted into a body lumen and configured to be expandable outward to restore a shape before being compressed when being indwelled in the body lumen; an outer tube configured to be capable of accommodating the self-expandable stent in an inner lumen thereof by being disposed on an outer surface side of the inner tube and discharging the self-expandable stent accommodated in the inner lumen by being moved toward the proximal side with respect to the inner tube; a pull wire configured to be capable of pulling the outer tube toward the proximal side; and an operation unit that operates advancing and retracting movement of the pull wire, wherein
the operation unit includes: a holding portion configured to hold the pull wire so that the pull wire is movable toward the distal side and toward the proximal side; and a switching portion configured to be capable of switching the pull wire held by the holding portion between limitation of movement toward the distal side and release of the limitation.

2. The self-expandable stent delivery system according to Claim 1, wherein the holding portion includes: a rotating member configured to be capable of moving rotationally and wind and feed the pull wire in association with a rotary movement, and
the switching portion includes an engaging portion configured to limit the rotary movement of the rotating member by engaging with the rotating member; and an engagement release portion configured to release the limitation by the switching portion by releasing the engagement between the rotating member and the engaging portion.

3. The self-expandable stent delivery system according to Claim 2, wherein the engagement release portion is configured to be capable of releasing the engagement along with a relative movement between the rotating member and the engaging portion away from each other.

4. The self-expandable stent delivery system according to Claim 3, further comprising:
an accommodation unit that accommodates the holding portion and the switching portion,
wherein the engagement release portion includes
a sliding member provided in the accommodation unit so as to be capable of advancing and retracting in a direction of advancing and retracting movement of the pull wire, and
a pressing member provided so as to be movable toward and away from the engaging portion and applying a pressing force in a direction to move the engaging portion away from the rotating member by moving toward the engaging portion in association with the advancing and retracting movement of the sliding member.

5. The self-expandable stent delivery system according to Claim 4 ,wherein the accommodation unit includes a first opening portion that exposes part of the holding portion to outside, a finger hooking portion on which a finger can be hooked when operating the pull wire via the holding portion, a second opening portion that exposes part of the engagement release portion at a position away from an extension line that connects the first opening portion and the finger hooking portion toward the proximal side.

6. The self-expandable stent delivery system according to any one of Claims 1 to 5, further comprising a locking portion that is capable of switching between restriction of the releasing operation of the switching portion for releasing the limitation and the releases of the restriction.
